# EUROPÄISCHE PATENTSCHRIFT

(11) **EP 0 654 994 B1**
(45) Veröffentlichungstag und Bekanntmachung des Hinweises auf die Patenterteilung: **25.11.1998**
(21) Anmeldenummer: 94918864.3
(22) Anmeldetag: 11.06.1994
(51) Int. Cl.: A61K 31/405, A61K 31/35, A61K 31/605

(54) **Verwendung von Cineol oder Menthol zur Behandlung von Asthma**
Use of cineol or menthol for the treament of asthma
Utilisation de cineol ou menthol pour le traitement d'asthme

(30) Priorität: 13.06.1993 DE 4319556; 13.06.1993 DE 4319554
(43) Veröffentlichungstag der Anmeldung: 31.05.1995
(73) Patentinhaber: JUERGENS, Uwe R., D-53859 Niederkassel (DE)
(72) Erfinder: JUERGENS, Uwe R., D-53859 Niederkassel (DE)
(74) Vertreter: Eggert, Hans-Gunther, Dr.
(86) Internationale Anmeldenummer: EP9401900
(87) Internationale Veröffentlichungsnummer: WO9428895

(56) Entgegenhaltungen:
- US-A- 4 775 667
- THERAPIEWOCHE, Bd.37, Nr.45, 1987 Seiten 4306 - 4311 H. GRIMM 'Antiobstruktive Wirksamkeit von Cineol bei Atemwegserkrankungen'
- PROSTAGLANDINS, Bd.20, Nr.2, 1980 Seiten 204 - 222 F. DEWHIRST 'Structure-activity relationshipsfor in hibition of prostaglandin cyclooxygenase by phenolic compounds'
- DATABASE MEDLINE US NATIONAL LIBRARY OF MEDICINE (NLM), BETHESDA, MD, US Knight-Ridder Information Nr. 07718086 Juni 1991, N. SIDELL ET AL. 'Retinoic acid-induced growth inhibition of a human myeloma cell line via down-regulation of Il-6 receptors' & J. IMMUNOL., Bd.146, Nr.11, Juni 1991
- BUNDESVERBAND DER PHARMAZEUTISCHEN INDUSTRIE E.V. 'rote liste 1992' 1992 , EDITIO CANTOR , AULENDORF/BRD siehe Nr. 28080: Rowachol comp.
- DATABASE MEDLINE US NATIONAL LIBRARY OF MEDICINE (NLM), BETHESDA, MD, US Knight-Ridder Information Nr. 07718086 Juni 1991, N. SIDELL ET AL. 'Retinoic acid-induced growth inhibition of a human myeloma cell line via down-regulation of Il-6 receptors' & J. IMMUNOL., Bd.146, Nr.11, Juni 1991

## Beschreibung

Akute und/oder chronisch-entzündliche oder akute allergische und/oder chronisch allergisch entzündliche Erkrankungen sind gekennzeichnet durch eine entzündliche Infiltration verschiedener Organsysteme durch Monozyten/Makrophagen, Eosinophile, Basophile und Neutrophile, Granulozyten, Mastzellen und Thrombozyten. Der Grad der entzündlichen Aktivität korreliert mit der Beeinflussung dieser Entzündungszellen in einem Organgewebe, in dessen Folge eine Schädigung des entsprechenden Organs verursacht wird. Diese Vorgänge sind bekannt bei primär entzündlich bedingten Erkrankungen der Atemwege, des Darms und des Gelencknorpels bei der rheumatoiden Arthritis. Da die primär auslösende Ursache bei den meisten chronisch-entzündlichen Erkrankungen nicht bekannt ist, können diese Erkrankungen nur durch eine unspezifische Suppression des Entzündungsreizes gebessert werden. Vor Auftreten von Krankheitssymptomen kann bei entsprechender genetischer Disposition oder in Gegenwart von Umweltnoxen die Reaktion des Organismus auf die exogene Noxe durch unspezifische Suppression des Entzündungsreizes prophylaktisch gehemmt werden.

Auf zellulärer Ebene kann das weitere Einwandern von Entzündungszellen in ein Entzündungsgebiet durch die Hemmung von chemotaktischen Faktoren vermindert werden, die ein Nachlassen der Entzündungsaktivität mit Abklingen der morphologischen und funktionellen Störungen des betroffenen Organsystems zur Folge hat. Diese Wirkung wird typischerweise durch immunsuppressive Agenzien, z.B. von Corticosteroiden, vermittelt. Diese Substanzgruppe ist bekannt durch ein Wirkprofil, das durch eine starke antiphlogistische Potenz die Entzündung hemmt, aber wegen der Verursachung schwergradiger Nebenwirkungen von Osteoporose, Magen- und Darmulcera, Steroidpurpura oder Lymphopenie nur schlecht vertragen wird.

Corticosteroide sind als Hemmstoffe der Phospholipaseaktivität und von Zytokinen in verschiedenen Entzündungszellen bekannt. Nach Inhibition der Phospholipaseaktivität wird die Freisetzung von Arachidonsäure aus den Phospholipidspeichern der Zellmembran gehemmt, die als wichtiges Substrat für die Bildung von verschiedenen Mediatoren bekannt ist.

Die eigentliche entzündungshemmende Wirkung wird durch Hemmung der Zytokinproduktion und über eine verminderte Bereitstellung von Arachidonsäure, des Präkusors für die Bildung von potenten chemotaktischen und an glatten Muskelzellen konstriktorisch wirksamen Metaboliten des 5-Lipoxygenasestoffwechselweges (Leukotriene) und des Cyclooxygenasestoffwechselweges (Prostaglandine, Thromboxan) vermittelt. Durch die anhaltende Suppression, insbesondere von Mediatoren des 5-Lipoxygenasestoffwechselweges wird deswegen der Bedarf systemisch-wirksamer Corticosteroide reduziert und bei nachlassender Infiltration mit Entzündungszellen die Aktivität des Entzündungsprozesses supprimiert.

Aus diesem Grunde kann durch eine unspezifische Entzündungshemmung der Krankheitsverlauf verschiedener akuter, chronischer oder allergischer Entzündungen günstig beeinflußt werden.

Gegenstand der Erfindung ist die Verwendung von 1,8-Oxydo-p-menthan, im folgenden kurz Cineol genannt, oder (-)Menthan-3-ol, im folgenden kurz Menthol genannt, zur Herstellung von Arzneimitteln in Form von magensaftresistenten Kapseln, Infusionslösungen oder Lösungen zur intramuskulären Injektion, zur Behandlung von Asthma bronchiale.

In Therapie-Woche 37, 4306 bis 4311(1987) beschreibt. H. Grimm eine Inhalationstherapie von Patienten mit chronischer Bronchitis oder Asthma. Dabei wird das Cineol nur topisch, d.h. lokal, zur rein symptomatischen, nichtkausalen Behandlung, insbesondere als Expektoranz, verwendet.
Die Verwendung von Cineol und/oder Menthol zur systemisch-kausalen entzündungshemmenden Behandlung von Asthma bronchiale durch systemische, z.B. perorale Gabe, insbesondere zur Reduktion systemisch wirksamer Corticosteroide ist nicht beschrieben.

Es wurde überraschenderweise gefunden, daß sich aus der Gruppe der Terpenstrukturen Cineol zur Behandlung von entzündlichen allergischen und/oder entzündlichen steroid-pflichtigen und infekt-exazerbierten broncho-pulmonalen Erkrankungen eignet, die die Bereitstellung von Arachidonsäure vermindert und auf diese Weise den Arachidonsäure-Metabolismus im 5-Lipoxygenasestoffwechselweg supprimiert. Außerdem wurde eine Hemmung von Interleukin 1β (IL₁-β) gefunden, das menschliche Zellen zur Freisetzung von Arachidonsäure konditioniert und selbst als starke Proinflammations-Mediator bekannt ist. Die Wirkungen sind möglicherweise auf seine lipophilen Eigenschaften, d.h. die Diffusion und Speicherung in Fettgeweben zurückzuführen. Dadurch ergibt sich eine neue Indikation für Cineol, insbesondere zur Therapie von entzündlichen oder entzündlich allergischen steroid-pflichtigen und infekt-exazerbierten Erkrankungen wie z.B. broncho-pulmonaler Erkrankungen. Beispielsweise wird durch die Wirkungsweise von Cineol der Gebrauch von Corticosteroiden in erheblichem Maße reduziert. Deshalb empfiehlt sich eine Dauertherapie.

Wegen des synergistischen Effektes empfiehlt sich eine kombinierte Therapie von Cineol mit den üblichen Corticosteroiden, Prednison, Prednisolon, Fluorcortolon, Beclomethason, Budesonid oder Flunisolid.

Cineol ist mit ca. 80 % der Hauptbestandteil der Eucalyptusöle vom Typ Eucalyptus Globulus verschiedener Arten. 1,8-Oxydomenthan ist ein bicyclischer Ether, der ein spannungsfreies Ringsystem besitzt und nach IUPAC 2-oxa-bicyclo /2,2,2/-octan genannt wird. Es handelt sich um eine farblose, würzig kampferähnlich riechende Flüssigkeit, die auch in Salbei-, Myrten-, Cajeputöl und anderen etherischen Ölen vorhanden ist.

Die Dosierung variiert bei den gesättigten mono- und bicyclischen Terpenstrukturen, liegt aber zwingend unter den Dosen, bei denen unerwünschte Nebenwirkungen oder gar Vergiftungen auftreten. So kann eine Dosierung von 200 bis 900 mg/Tag, vorzugsweise 600 mg/Tag, in Form magensaftresistenter Kapseln mit einer Dosiseinheit von 100 mg/Kapsel verabreicht werden. 1,8-Cineol und Menthol sind geeignet als Zusätze zur intravenösen Dauerinfusion und zur intramuskulären Applikation. Cineol und Menthol können mit anderen Substanzen kombiniert werden.

Die Dosierung für Menthol liegt zwischen 100 und 450 mg/Tag. Cineol wird exemplarisch für die Zwecke der Erfindung in einer Dosierung von 200 bis 900 mg/Tag, vorzugsweise 600 mg/Tag verabreicht. Die Gesamtmenge verteilt sich für die genannten Substanzen zweckmäßig über eine dreimalige Einnahme pro Tag.

Die medizinischen Zubereitungsformen können fest oder flüssig sein. Außerdem besteht die Möglichkeit, den Wirkstoff mit den in der Galenik üblichen, pharmakologisch unbedenklichen und mit -Cineol verträglichen Träger, Verdünnungs- und Zusatzmitteln zu verabreichen. Zusatzmittel sind u.a. Füllstoffe, Spreng-, Binde-, Netz-, Stabilisierungs-, Gleit-, Emulgier-, Süß-, Geschmacksmittel und ähnliche. Zu diesen Zusatzmitteln gehören z.B. Melantinlösungen, Pektinlösungen, Milchzucker, Kochsalz, Talkum, Stärke, Borsäure, Paraffinöl, Paraffine, Stearinsäure und deren Derivate, Kakaobutter, Gummi, Sirupe, Süßholzextrakte, Hefeextrakte, Honig, Glycerin, Kieselgur, Kaolin, Magnesiumoxid, Bienenwachs und pflanzliche Öle.

Bei flüssigen Zubereitungsformen können Wasser, Glycerin, Zucker- oder Alkohollösungen oder Mischungen solcher als Träger und Hilfsstoff verwendet werden. Die Methoden der Rezepturoptimierung sind dem Fachmann bekannt (Chemie in unserer Zeit 23, 114 und 161 (1989)).

### BEISPIEL 1

Durch in-vitro-Untersuchungen wurde gefunden, daß die Wirkung von Cineol auf der Hemmung der Arachidonsäure-Freisetzung aus den Phospholipidspeichern in nahezu allen menschlichen Zellen basiert mit der Folge, daß die Arachidonsäure als Substrat für den 5-Lipoxygenase- und Cyclooxygenase-Stoffwechselweg in nur vermindertem Maße für die Mediatorbildung zur Verfügung steht.

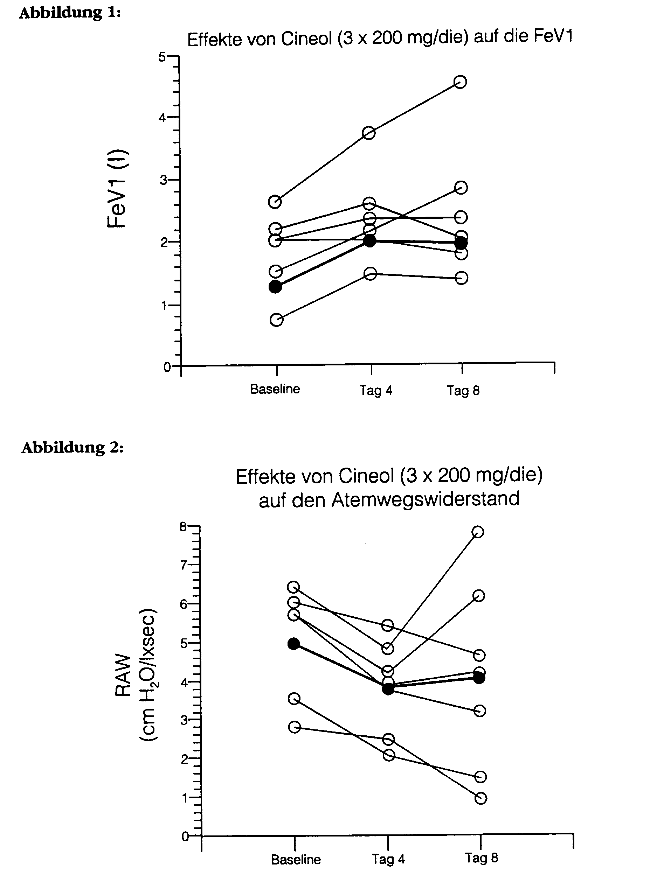

In diesen Versuchen konnte nach viertägiger Therapie von Patienten mit allergischem und nicht allergischem Asthma bronchiale (n = 7) eine signifikante Suppression der monozytären LTB₄-Produktion ex vivo unter Verbesserung der Lungenfunktion nachgewiesen werden (s. Tabelle I, Abb. 1 - 4). Patienten mit allergischem und nicht allergischem Asthma bronchiale wurde jeweils 50 ml Venenblut vor Therapie, nach 4-tägiger Therapie und 4 Tage nach Absetzen von Cineol (Tag 8) abgenommen. Daraus wurden mittels einer Dichte-Gradient-Zentrifugation Monozyten isoliert und durch weitere Zentrifugation gereinigt. Jeweils 50000 Zellen wurden in einem Milliliter Kulturüberstand in Plastikröhrchen über 30 Minuten mit Calcium Ionophor A23187 in einem Wasserbad bei 37 °C stimuliert. Nach der Inkubation wurden die Röhrchen mit den Zellen 5 Minuten lang bei 4 °C zentrifugiert und die Kulturüberstände gewonnen und bei -80 °C bis zur weiteren Analyse gelagert. Anschließend wurden die Kulturüberstände aufgetaut und LTB₄ durch einen spezifischen Enzym-Immuno-Assay bestimmt.

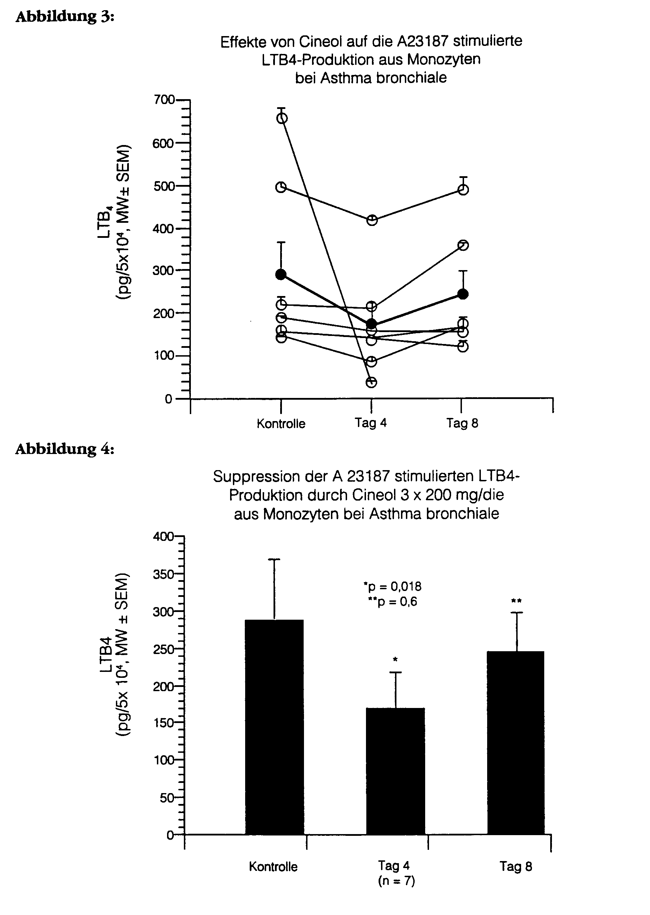

Nach 4-tägiger Therapie der gleichen sieben Patienten mit 3 x 200 mg Cineol in Kapseln wurde Blut abgenommen und die gleiche Analyse auf LTB₄ wiederholt. Dann wurde die Behandlung mit Cineol abgesetzt und die gleiche Untersuchung nach weiteren 4 Tagen (Tag 8) wiederholt. Die Ergebnisse sind in den Abbildungen 3 und 4 wiedergegeben. Wie die beiden Abbildungen zeigen, bewirkt Cineol in allen Fällen eine signifikante Abnahme der LTB₄-Produktion, die aber nach Absetzen des Cineols wieder auf den Ausgangswert angestiegen ist (Tag 8). Die Klinischen Daten zeigt die Tabelle I in Verbindung mit den Abbildungen 1 und 2, wobei FeV1 die forcierte 1-Sekunden-Kapazität und RAW den Atemwegswiderstand bedeutet.

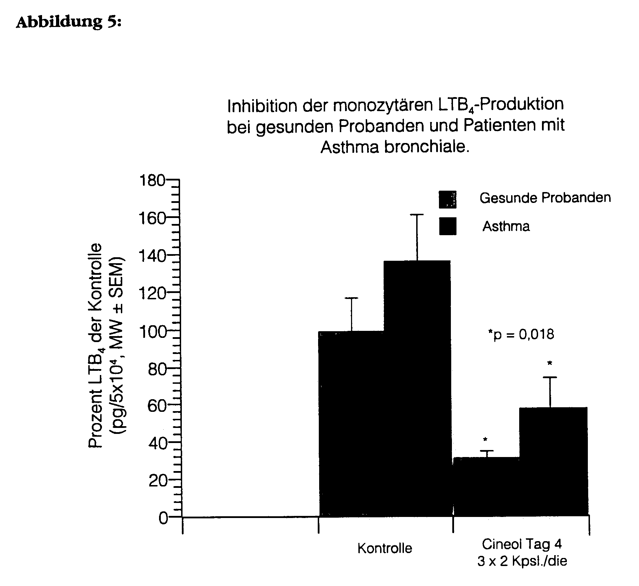

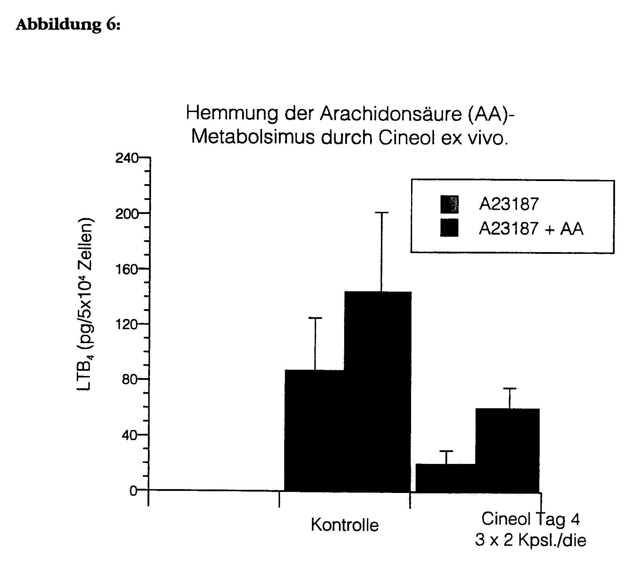

Zum Vergleich wurde auch die LTB₄-Produktion in Monozyten von sieben gesunden Probanden (25 ± 2 Jahre) vor und nach der gleichen Cineol-Behandlung untersucht (s. Abb. 5). Im Vergleich zu Patienten mit Asthma war die LTB₄-Produktion auch bei gesunden Patienten abgefallen, so daß von einer generellen Wirkung auszugehen ist. Die Abbildung 6 zeigt, daß auch Cineol allein die Calcium Ionophor A23187-stimulierte LTB₄-Produktion in Monozyten inhibiert und sich infolgedessen auch allein zur Behandlung von entzündlichen infekt-exazerbierten broncho-pulmonalen Erkrankung eignet.

Die rechten Säulen der Abbildung 5 zeigen die unerwartete synergistische Wirksamkeit von Cineol der erfindungsgemäßen Behandlung bei Patienten mit Asthma bronchiale, weil selbst die Cortison-Behandlung die LTB₄-Produktion nicht auf das Normalmaß reduzieren konnte, während die gleichzeitige Cineol-Behandlung die LTB₄-Produktion in den Normalbereich bringt und dadurch in gleichem Maße Cortison eingespart werden kann.

Das gleiche beweist eine Langzeit-Therapie (43 ± 7 Tage) von Patienten mit steroid-pflichtigem Asthma bronchiale (n = 5), die unter Cineol (3 x 200 mg/die) von einer signifikanten Abnahme des Steroidbedarfs und einer Verbesserung der Lungenfunktion profitieren (Tabelle II). Unter Dauertherapie mit Cineol ist die A23187 stimulierte LTB₄-Produktion aus Monozyten ex vivo auch nach Reduktion der systemischen Glukocorticosteroidtherapie nicht angestiegen.

Der Corticosteroidbedarf für Cineol betrug durchschnittlich 8,9 ± 3,3 mg/Tag (Bereich 0 - 24 mg) und konnte nach 43,5 ± 7,3 Tagen bis auf 4,0 ± 1,3 mg/Tag (Bereich 0 - 8 mg/Tag) reduziert werden. Die Reduktion der Steoridtherapie hatte keinen negativen Einfluß auf die Lungenfunktion, die FeV₁ vor Cineol auf 1,83 ± 0,26 Liter nach Langzeittherapie (für n = 6 Probanden) auf 2,7 ± 0,55 Liter angestiegen (p = 0,0431). Die Resistenz vor der Cineol-Behandlung (5,49 ± 1,02 cm H₂O/Liter x Sekunden) war nach Dauertherapie mit Cineol auf 3,98 + 1,2 cm H₂O pro 1 x Sekunden abgefallen (p = 0,0273). Die LTB₄-Produktion für Cineol war nach der Dauertherapie ebenfalls abgefallen. Die Langzeittherapieergebnisse zeigen erstmalig, daß unter Therapie mit Cineol der tägliche Steroidbedarf deutlich abnimmt, ohne daß eine Verschlechterung der Lungenfunktion eintritt. Die durchschnittliche LTB₄-Produktion unter Cineol nach der Langzeittherapie ist deutlich supprimiert. Die Reduktion der systemischen Corticosteroide führt unter Langzeittherapie mit Cineol nur zu einem Anstieg der LTB₄-Produktion bei Patienten, die durch hohe Steroiddosis eine vor Cineol supprimierten LTB₄-Produktion in vitro demonstriert hatten.

Die Untersuchung der Lipopolysaccharid(LPS)-stimulierten Bildung von IL₁-β aus Monozyten von gesunden Probanden (n = 4) wurde vor Einnahme von Cineol nach viertägiger Therapie mit 3 x 2 Kapseln (= 3 x 200 mg Cineol/Tag) und 4 Tage nach Absetzen von Cineol bestimmt ( Tag 8). In allen untersuchten Probanden wurde die IL₁-β Produktion am Tag 4 und 8 um durchschnittlich 60 % gehemmt. Da die Produktion von IL₁-β auch noch 4 Tage nach Absetzen von Cineol (= Tag 8) supprimiert war, ist von einer starken antiphogistischen Wirkung auszugehen, die wegen der Langzeitwirkung auch zur Prophylaxe, z.B. bei Patienten mit Asthma bronchiale und Kindern sehr geeignet ist.

### Beispiel 2

Gesunde Probanden wurden 4 Tage mit Menthol 3 x 150 mg/Tag (Dosiseinheit = 150 mg/Kapsel) per os behandelt. Nach viertägiger Therapie war die Calcium-Ionophor A23187 stimulierte LTB₄-Produktion isolierter Monozyten bis zu 75 % supprimiert.

Bei Patienten mit Asthma bronchiale konnte wie unter Therapie mit Cineol nach viertägiger Therapie in obiger Dosis eine Verbesserung der Lungenfunktion erreicht werden. Nach zwölfwöchiger Dauertherapie hatte sich das Bronchialasthma so stabilisiert, daß der systemische Corticosteroidbedarf um bis zu 50 % reduziert werden konnte. Die Therapie wurde ohne Nebenwirkungen gut vertragen.

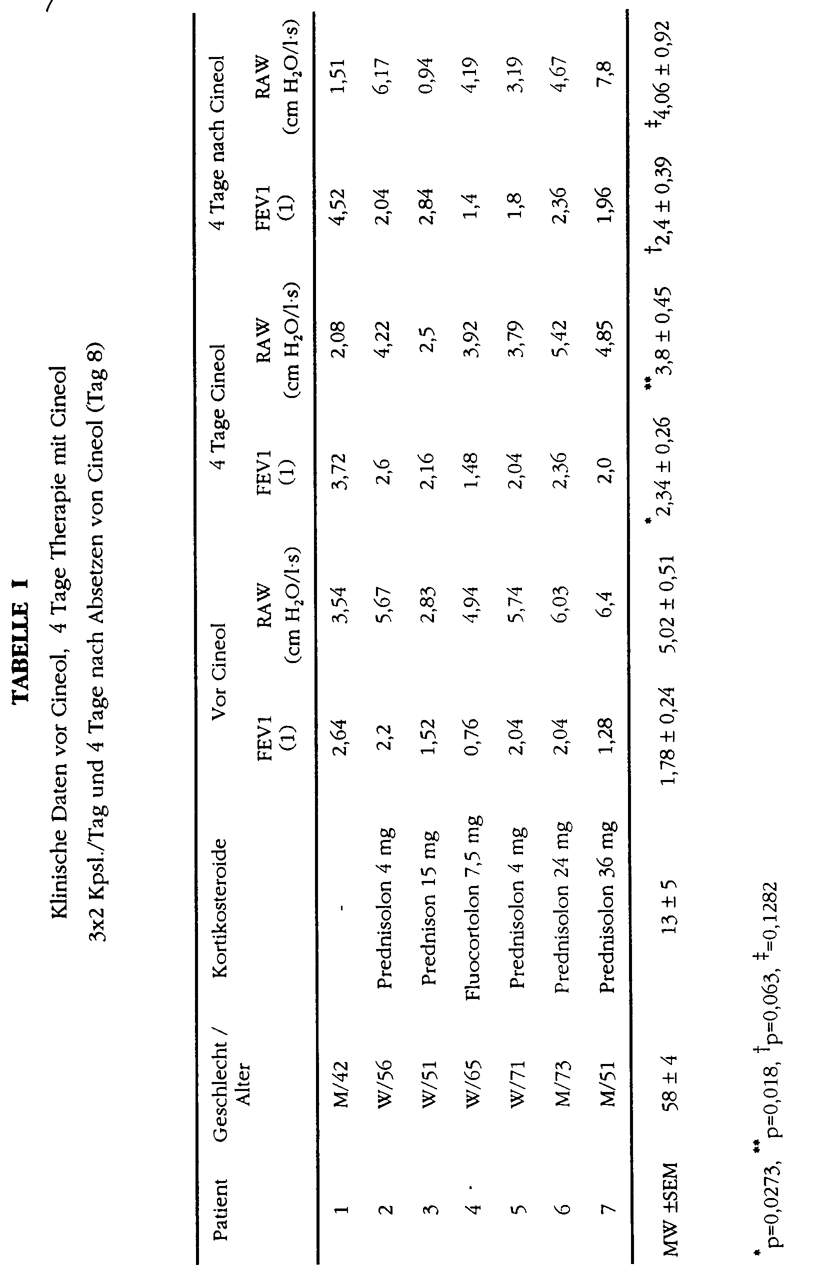

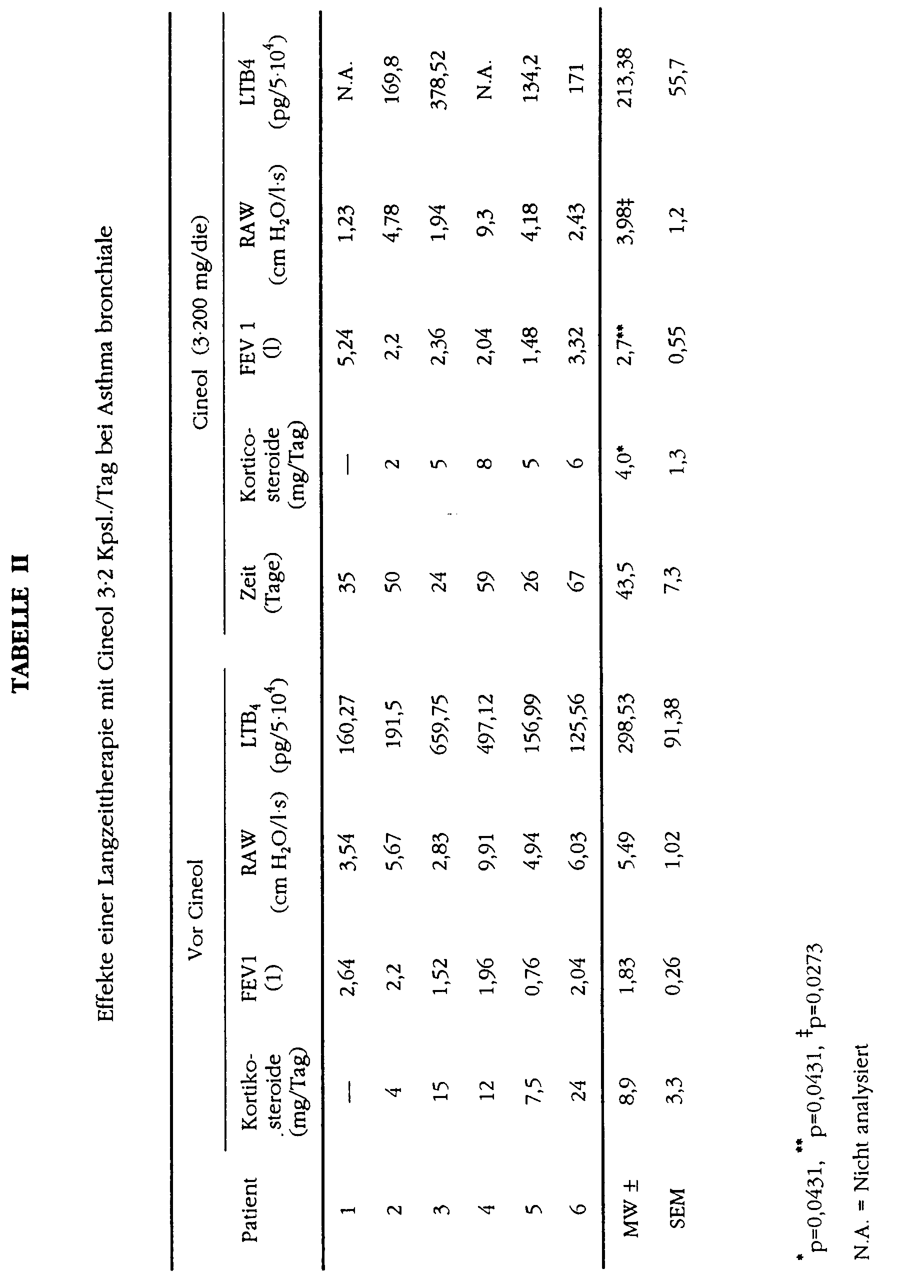

## Patentansprüche

1. Verwendung von 1,8-Oxydo-p-menthan oder Menthan-3-ol zur Herstellung von Arzneimitteln in Form von magensaftresistenten Kapseln, Infusionslösungen oder Lösungen zur intramuskulären Injektion, zur Behandlung von Asthma bronchiale.

2. Verwendung nach Anspruch 1 zur Reduktion systemisch wirksamer Corticosteroide.

3. Verwendung nach Anspruch 1 oder 2 zur Monotherapie bei mildem oder leichtem Asthma bronchiale sowie zur Behandlung von Kindern mit Asthma.

4. Verwendung nach einem der Ansprüche 1 bis 3 zur Prophylaxe.

5. Verwendung nach Anspruch 1, 2 oder 4 in Kombination mit systemisch wirksamen Medikamenten, insbesondere Immunsuppressiva oder Corticosteroiden.

6. Verwendung nach einem der Ansprüche 1 bis 5, dadurch gekennzeichnet, daß die zu verabreichbarende tägliche Gesamtmenge an 1,8-Oxydo-p-menthan oder Menthan-3-ol im Bereich von 100 bis 900 mg liegt.

7. Verwendung von 1,8-Oxydo-p-menthan für die Zwecke des Anspruchs 1 in einer zu verabreichbaren täglichen Gesamtmenge zwischen 200 und 900 mg, vorzugsweise 600 mg.

8. Verwendung von Menthan-3-ol für die Zwecke des Anspruchs 1 in einer zu verabreichbaren täglichen Gesamtmenge zwischen 100 und 450 mg.

9. Verwendung nach einem der Ansprüche 1 bis 8, dadurch gekennzeichnet, daß die Dosiseinheit für die orale Therapie in Form von magensaftresistenten Kapseln 100 mg beträgt.

## Claims

1. Use of 1,8-oxido-p-menthane or menthane-3-ol for production of drugs in the form of capsules resistant to gastric juice, infusion solutions or as solutions for intramuscular injection for treatment of bronchial asthma.

2. Use according to claim 1 for the reduction of systemically active corticosteroids.

3. Use according to claim 1 or 2 for monotherapy in mild or slight bronchial asthma as well as for treatment of children with asthma.

4. Use for prophylaxis according to one of the claims 1 to 3.

5. Use according to claims 1, 2 or 4 in combination with systemically active drugs, especially immunosuppressives or corticosteroids.

6. Use according to one of the claims 1 to 5 characterized by a total daily intake amount of 1,8-oxido-p-menthane or menthane-3-ol in the range from 100 mg to 900 mg.

7. Use of 1,8-oxido-p-menthane for the purposes of claim 1 in a total daily intake amount between 200 mg and 900 mg, preferably 600 mg.

8. Use of methane-3-ol for the purposes of claim 1 in a total daily intake amount between 100 mg and 450 mg.

9. Use according to one of the claims 1 to 8 characterized by a dose unit for oral therapy in the form of capsules resistant to gastric juice of 100 mg.

## Revendications

1. Utilisation d'1,8-oxydo-p-menthane où menthan-3-ol pour la fabrication de produits pharmaceutiques sous forme de capsules résistantes au suc gastrique, solutions en infusion où solutions pour l'injection intramusculaire pour le traitement d'asthme bronchique.

2. Utilisation selon revendication 1 pour la réduction de corticostéroïdes systémiquement actifs.

3. Utilisation selon revendication 1 où 2 pour monothérapie pour l'asthme bronchique doux où léger ainsi que pour le traitement d'enfants asthmatiques.

4. Utilisation selon l'une des revendications 1 à 3 pour la prophylaxie.

5. Utilisation selon revendication 1, 2 ou 4 en combination avec des médicaments systémiquement actifs, particulièrement immunosuppressiva où corticostéroïdes.

6. Utilisation selon l'une des revendications 1 à 5, caractérisée en ce que la quantité totale a administrer journalièrement d'1,8-oxydo-p-menthane où menthan-3-ol est comprise entre 100 et 900 mg.

7. Utilisation d'1,8-oxydo-p-menthane dans l'objectif de la revendication 1 dans une quantité totale a administrer journalièrement comprise entre 200 et 900 mg de préférence 600 mg

8. Utilisation de menthan-3-ol dans l'objectif de la revendication 1 dans une quantité totale a administrer journalièrement comprise entre 100 et 450 mg.

9. Utilisation selon l'une des revendications 1 à 8, caractérisée par une dose unitaire pour la thérapie orale sous forme de capsules résistantes au suc gastrique est de 100 mg
